# EUROPÄISCHE PATENTANMELDUNG

(11) **EP 4 810 469 A1**
(43) Veröffentlichungstag der Anmeldung: **23.09.2026**
(21) Anmeldenummer: 25164637.8
(22) Anmeldetag: 19.03.2025
(51) Int. Cl.: C07C 45/50, C07C 47/02, C07C 47/21, C07F 9/6574, C07F 15/00

(54) **LIGANDEN FÜR ORGANOPHOSPHITKATALYSATOREN**

(71) Anmelder: Evonik Oxeno GmbH & Co. KG, 45772 Marl (DE)
(72) Erfinder: FRANKE, Robert, 45772 Marl (DE); BÜKER, Julia, 44803 Bochum (DE); SALE, Anna Chiara, 40474 Düsseldorf (DE); BÖRNER, Armin, 18059 Rostock (DE); Holz, Jens, 18196 Kessin (DE); ROMEIKE, Kerstin, 18109 Rostock (DE); WENZEL, Gudrun, 18196 Kessin (DE)
(74) Vertreter: Evonik Patent Association

(57) **Zusammenfassung**

Die Erfindung betrifft Verbindungen in Form von Organobisphosphiten der Formel (I) mit ausgewählten Strukturelementen (i), (ii), (iii), (iv), (v) und (vi). Die Erfindung betrifft zudem die Verwendung der Verbindungen als Ligand eines Katalysators in einer Hydroformylierungsreaktion. Die vorliegende Erfindung betrifft zudem eine Substanz umfassend eine Verbindung der Formel (I) und die Verwendung dieser Substanz als Katalysator in einer Hydroformylierungsreaktion. Die vorliegende Erfindung betrifft darüber hinaus ein katalytisches Verfahren zur Herstellung von Aldehyden.

## Beschreibung

Die vorliegende Erfindung betrifft eine Verbindung gemäß Formel (I) und die Verwendung der Verbindung als Ligand eines Katalysators in einer Hydroformylierungsreaktion. Die vorliegende Erfindung betrifft zudem eine Substanz und die Verwendung dieser Substanz als Katalysator in einer Hydroformylierungsreaktion. Die vorliegende Erfindung betrifft darüber hinaus ein Verfahren zur Herstellung von Aldehyden.

Die Hydroformylierung (auch Oxosynthese oder Roelen-Reaktion genannt) war die erste großtechnisch genutzte und heutzutage mit über 10 Mio. t/a mengenmäßig wichtigste Synthese, welche mit metallorganischen Katalysatoren durchgeführt wurde/wird. Bei der Hydroformylierung reagiert ein Synthesegas aus Wasserstoff und Kohlenstoffmonoxid mit Kohlenstoff-Doppelbindungen von Olefinen unter Bildung von Aldehyden; die Reaktion wird vorzugsweise durch Cobalt- oder Rhodiumkatalysatoren katalysiert.

Durch die Auswahl der Komponenten, insbesondere durch elektronische und sterische Eigenschaften von Metallatom und Liganden des metallorganischen Katalysators, kann die Hydroformylierung von Olefinen gesteuert und optimiert werden. Insbesondere kann durch die gezielte Auswahl der Liganden in metallorganischen Katalysatoren der Umsatz und auch die Selektivität gesteigert werden.

Als Liganden des Katalysators spielen insbesondere die Organobisphosphite eine herausragende Rolle (siehe R. Franke, D. Selent, A. Börner Chem. Rev. 2012, 112, 11, 5675-5732, https://doi.org/10.1021/cr3001803).

Das Dokument EP 0 577 042 A1 offenbart ein Hydroformylierungsverfahren zur Herstellung eines 1,6-Hexandials.

Die als Ligand eingesetzten Verbindungen sollen hierbei insbesondere einen hohen Umsatz der Olefine in Aldehyde gewährleisten.

Puh kDemnach liegt der vorliegenden Erfindung die primäre Aufgabe zu Grunde, neue Verbindungen herzustellen, die als Liganden in Katalysatorsystemen für die Hydroformylierung geeignet sind.

Entsprechend basiert die vorliegende Erfindung auch auf der Aufgabe, eine Substanz enthaltend die zuvor genannten Verbindungen anzugeben, welche katalytisch aktiv ist.

Die vorliegende Erfindung basiert zudem auf der Aufgabe, ein (katalytisches) Verfahren zur Herstellung von Aldehyden aus Olefinen anzugeben, wobei insbesondere der Umsatz optimiert werden soll.

Diese Aufgaben sind eng verknüpft mit der ergänzenden Aufgabenstellung, eine Verwendung für die zuvor genannten Verbindungen und/oder die zuvor genannten Substanzen anzugeben.

Weitere Aufgaben ergeben sich aus der nachfolgenden Beschreibung und den Patentansprüchen.

Der Gegenstand der vorliegenden Erfindung wird in den beigefügten Patentansprüchen und der vorliegenden Beschreibung definiert.

Die vorliegende Erfindung betrifft insbesondere eine Verbindung gemäß Formel (I), 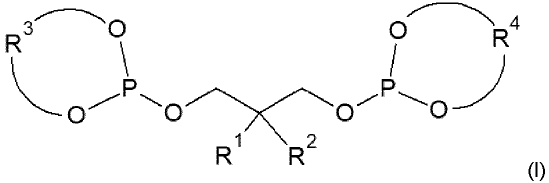 wobei
- R¹ und R² jeweils unabhängig voneinander ausgewählt sind aus: H und CH₃
   und
- R³ und R⁴ jeweils unabhängig voneinander ausgewählt sind aus den Strukturelementen (i), (ii), (iii), (iv), (v) und (vi)

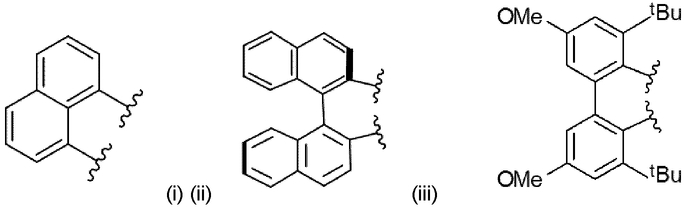

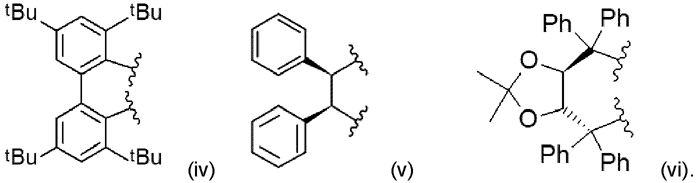

Jede durch eine Schlängellinie markierte Bindung der Strukturelemente (i), (ii), (iii), (iv), (v) und (vi) verknüpft das zur markierten Bindung gehörende C-Atom mit einem der in Formel (I) dargestellten Sauerstoffatome, welche mit den Substituenten R³ oder R⁴ direkt verbunden sind.

Die erfindungsgemäßen Verbindungen konnten erstmalig synthetisiert werden und haben sich in eigenen Untersuchungen als besonders vorteilhafte Liganden zur katalytischen Herstellung von Aldehyden aus Olefinen in einer Hydroformylierungsreaktion herausgestellt.

Die erfindungsgemäßen Verbindungen gemäß Formel (I) sind Organobisphosphite (oft auch als "Bisphosphite" bezeichnet). Ein Phosphit hat die allgemeine Form P(OR)₃ mit (R = Alkyl-Rest oder Aryl-Rest). Durch geschickte Wahl einer strukturell flexiblen linearen oder verzweigten Alkylkette als Bindeglied der beiden Phosphitgruppen und durch weiterhin geschickte Wahl der Strukturelemente (i), (ii), (iii), (iv), (v) und (vi) als verbrückte Doppelsubstituenten der Phosphitgruppen, konnten im Rahmen der vorliegenden Erfindung für die Hydroformylierung besonders gut geeignete Liganden bereitgestellt werden. Die erfindungsgemäßen Verbindungen gemäß Formel (I) weisen zwei potentielle Bindungsstellen zu einem Metallzentralatom auf, liegen also in Form zweizähniger Liganden vor, was die daraus resultierende Metallkomplexe durch den sogenannten Chelateffekt besonders stabil und robust macht.

Die im Rahmen der vorliegenden Erfindung durchgeführten Untersuchungen, vgl. Auch die unten gezeigten Beispiele, zeigen, dass mit den erfindungsgemäßen Organobisphosphit-Liganden deutlich bessere Umsätze bei der Synthese von Aldehyden aus Olefinen in einer Hydroformylierungsreaktion erzielt werden können, als mit bekannten Systemen, insbesondere bessere Umsätze als mit Organobisphosphinit-Liganden (oft auch als "Bisphosphinit" bezeichnet).

Durch Verwendung der erfindungsgemäßen Verbindungen als Ligand in einem Katalysator konnte in Hydroformylierungsreaktionen von Olefinen ein Umsatz von über 80 % erreicht werden.

Des Weiteren zeigen die mit diesen Liganden hergestellten Katalysatoren die vorteilhafte Eigenschaft interne Olefine (zum Beispiel 2-Penten) in einem nicht unerheblichen Anteil zu terminalen Aldehyden zu hydroformylieren, sogenannte n-Selektivität.

Bevorzugt ist eine erfindungsgemäße Verbindung gemäß Formel (I), wobei R³ und R⁴ jeweils unabhängig voneinander ausgewählt sind aus den Strukturelementen (i), (ii), (iv), (v) und (vi).

Ebenfalls bevorzugt ist eine erfindungsgemäße Verbindung gemäß Formel (I), wobei R³ und R⁴ jeweils unabhängig voneinander ausgewählt sind aus den Strukturelementen (i), (ii),(iii), (v) und (vi).

Besonders bevorzugt ist eine erfindungsgemäße Verbindung gemäß Formel (I), wobei R³ und R⁴ jeweils unabhängig voneinander ausgewählt sind aus den Strukturelementen (ii), (v) und (vi). Mit diesen Ligandensystemen können besonders hohe Umsätze in der Hydroformylierung von 2-Penten von ≥ 94% erreicht werden.

Bevorzugt ist eine erfindungsgemäße Verbindung (vorzugsweise wie vorstehend als bevorzugt bezeichnet), wobei
- R¹ und R² jeweils H sind,
   oder
- R¹ und R² jeweils CH₃ sind.

Interne Untersuchungen haben gezeigt, dass besonders hohe Umsätze in Hydroformylierungsreaktionen von Olefinen in Aldehyde erreicht werden konnten, wenn die Reste R¹ und R² identisch sind.

Bevorzugt ist eine erfindungsgemäße Verbindung (vorzugsweise wie vorstehend als bevorzugt bezeichnet), wobei R³ und R⁴ identisch sind und jeweils
- das Strukturelement (i) sind,
   oder
- das Strukturelement (ii) sind,
   oder
- das Strukturelement (iii) sind,
   oder
- das Strukturelement (iv) sind,
   oder
- das Strukturelement (v) sind,
   oder
- das Strukturelement (vi) sind.

Interne Untersuchungen haben gezeigt, dass besonders hohe Umsätze erreicht werden konnten, wenn die Reste R³ und R⁴ identisch sind.

Bevorzugt sind R³ und R⁴ identisch und jeweils das Strukturelement (i) oder das Strukturelement (ii) oder das Strukturelement (iv) oder das Strukturelement (v) oder das Strukturelement (vi).

Bevorzugt sind R³ und R⁴ identisch und jeweils das Strukturelement (i) oder das Strukturelement (ii) oder das Strukturelement (iii) oder das Strukturelement (v) oder das Strukturelement (vi).

Bevorzugt sind R³ und R⁴ identisch und jeweils das Strukturelement (i) oder das Strukturelement (ii) oder das Strukturelement (v) oder das Strukturelement (vi).

Weiter bevorzugt sind R³ und R⁴ identisch und jeweils das Strukturelement (ii) oder das Strukturelement (v) oder das Strukturelement (vi).

Besonders bevorzugt sind R³ und R⁴ identisch und jeweils das Strukturelement (ii) oder das Strukturelement (v).

Mit einer Verbindung als Ligand, in der R³ und R⁴ jeweils das Strukturelement (vi) waren, konnte bei der Hydroformylierung von 2-Penten ein Umsatz von 94% erreicht werden.

Mit einer Verbindung als Ligand, in der R³ und R⁴ jeweils das Strukturelement (ii) waren, konnte bei der Hydroformylierung von 2-Penten ein Umsatz von 99% erreicht werden.

Mit einer Verbindung als Ligand, in der R³ und R⁴ jeweils das Strukturelement (v) waren, konnte bei der Hydroformylierung von 2-Penten ein Umsatz von >99% erreicht werden.

Bevorzugt ist eine erfindungsgemäße Verbindung (vorzugsweise wie vorstehend als bevorzugt bezeichnet), ausgewählt aus der Gruppe von Verbindungen mit den folgenden Formeln (I-i), (I-ii), (I-iii), (I iv), (I-v), (I-v-Di-Me), (I-v-Di-H), oder (I-vi): 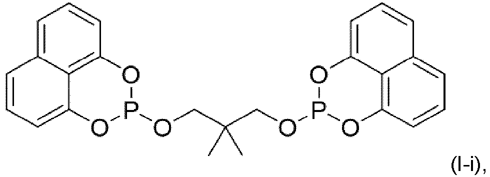 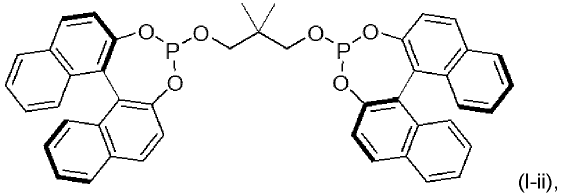 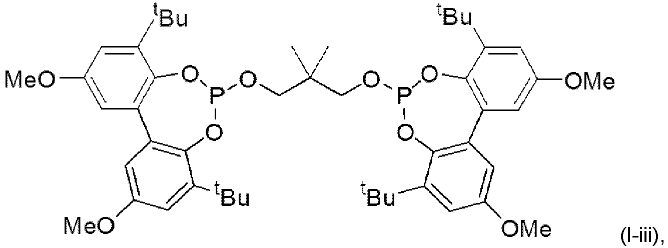 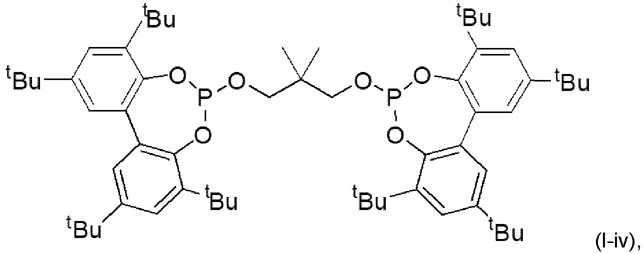 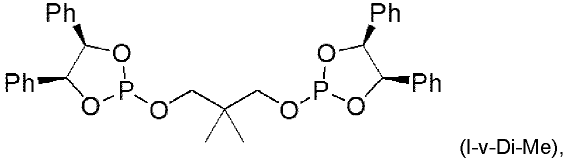 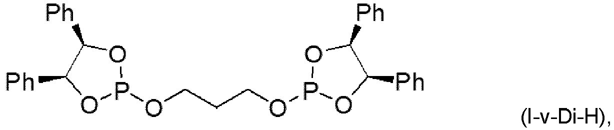 oder

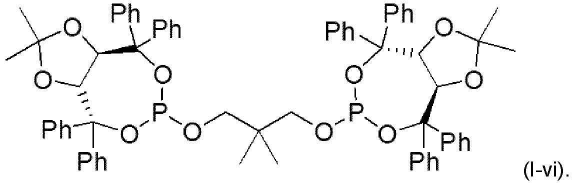

Bevorzugt ist die Verbindung ausgewählt aus den Verbindungen I-i, I-ii, I-iv, I-v-Di-Me, I-v-Di-H und I-vi.

Bevorzugt ist die Verbindung ausgewählt aus den Verbindungen I-i, I-ii, I-iii, I-v-Di-Me, I-v-Di-H und I-vi.

Bevorzugt ist die Verbindung ausgewählt aus den Verbindungen I-i, I-ii, I-v-Di-Me, I-v-Di-H und I-vi.

Weiterhin bevorzugt ist die Verbindung ausgewählt aus den Verbindungen I-ii, I-v-Di-Me, I-v-Di-H und I-vi.

Mit den Verbindungen I-ii, I-v-Di-Me, I-v-Di-H und I-vi als Liganden eines Katalysators konnten bei der Hydroformylierung von 2-Penten hohe Umsätze von ≥94% erhalten werden.

Besonders bevorzugt ist die Verbindung ausgewählt aus den Verbindungen I-ii, I-v-Di-Me und I-v-Di-H.

Mit den Verbindungen I-ii, I-v-Di-Me und I-v-Di-H als Liganden eines Katalysators konnten bei der Hydroformylierung von 2-Penten sehr hohe Umsätze von ≥99% erzielt werden.

Ganz besonders bevorzugt ist die Verbindung ausgewählt aus den Verbindungen I-v-Di-Me und I-v-Di-H.

Mit den Verbindungen I-v-Di-Me und I-v-Di-H als Ligand eines Katalysators konnten bei der Hydroformylierung von 2-Penten exzellente Umsätze von >99% erhalten werden.

Die vorliegende Erfindung betrifft zudem eine Substanz, wobei die Substanz
i) eine Mischung ist, die Mischung umfassend
   - eine oder mehrere erfindungsgemäße Verbindungen (vorzugsweise wie vorstehend als bevorzugt bezeichnet),
      und
   - eines oder mehrere Metallatome,
      und/oder
ii) ein Metallkomplex ist oder einen Metallkomplex enthält, der Metallkomplex umfassend
   - eine oder mehrere erfindungsgemäße Verbindungen (vorzugsweise wie vorstehend als bevorzugt bezeichnet),
      und
   - eines oder mehrere Metallatome.

Erfindungsgemäße Substanzen in Form von Mischungen oder Metallkomplexen wie vorstehend und nachstehend definiert eignen sich insbesondere als Katalysatorsysteme in Hydroformylierungsreaktionen.

In erfindungsgemäßen Substanzen sind neben den erfindungsgemäßen Verbindungen zusätzlich eines oder mehrere Metallatome umfasst. Die erfindungsgemäßen Verbindungen, d.h. die Liganden, und die Metallatome (vor der Komplexbildung oft auch als "Metallprecursor" bezeichnet) bilden bevorzugt einen Metallkomplex, der katalytisch aktiv ist, insbesondere in einer Hydroformylierungsreaktion.

Der Metallkomplex kann *in situ* durch Zusammengeben des erfindungsgemäßen Liganden und eines Metallprecursors erzeugt werden (Variante i).

Der Metallkomplex mit dem entsprechenden Liganden kann jedoch auch gesondert hergestellt, isoliert und weiterverwendet, insbesondere als Katalysator eingesetzt werden, oder an Dritte verkauft werden (Variante ii).

Bevorzugt ist eine erfindungsgemäße Substanz, wobei das molare Verhältnis von Metallatomen zu Verbindungen gemäß Formel (I), d.h. den Liganden, in der Mischung und/oder im Metallkomplex im Bereich von 0.5:1 bis 1:4 liegt, bevorzugt im Bereich von 0.9:1 bis 1:2.1 liegt, besonders bevorzugt 1:1 oder 1:2 ist.

Ebenfalls bevorzugt ist eine erfindungsgemäße Substanz, wobei das eine oder eines der mehreren Metallatome in der Mischung und/oder im Metallkomplex ausgewählt ist aus der Gruppe bestehend aus Rhodium, Cobalt, Eisen, Ruthenium, Osmium, Iridium, Platin und Zinn, bevorzugt ist das eine oder eines der mehreren Metallatome Rhodium.

Bevorzugt ist das eine oder eines der mehreren Metallatome im Rahmen der vorliegenden Erfindung Cobalt oder Rhodium. Cobalt und Rhodium haben sich als Metallatom in Katalysatoren für die Hydroformylierung als besonders geeignet erwiesen.

Ebenfalls bevorzugt ist eine erfindungsgemäße Substanz in Form einer Mischung, wobei die Mischung als weiteren Bestandteil umfasst
- ein oder mehrere Olefine mit jeweils einer oder mehreren Kohlenstoff-Doppelbindungen, bevorzugt ausgewählt aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
   und/oder (bevorzugt "und)
- Lösungsmittel, wobei das Lösungsmittel vorzugsweise Toluol umfasst oder Toluol ist.

Vorgenannte bevorzugte Ausgestaltungen der erfindungsgemäßen Substanz in Form einer Mischung, zusätzlich umfassend eines oder mehrere Olefine mit jeweils einer oder mehreren Kohlenstoff-Doppelbindungen können direkt in einer Hydroformylierungsreaktion eingesetzt werden. Sie umfassen sowohl einen wie zuvor beschriebenen Katalysator als auch eine oder mehrere olefinische Ausgangsverbindungen. Bevorzugt umfasst die Mischung ebenfalls ein geeignetes Lösungsmittel, insbesondere Toluol, um die Reaktionsführung zu erleichtern.

In einer Hydroformylierungsreaktion findet formal die Addition eines Wasserstoffs (-H) und einer Formylgruppe (-CHO) an eine Kohlenstoff-Doppelbindung eines Moleküls statt; somit entstehen Aldehyde.

Diese Kohlenstoff-Doppelbindungen können sowohl in internen Olefinen wie zum Beispiel in 2-Penten vorliegen, als auch in endständigen Olefinen wie zum Beispiel in 1,3-Butadien vorliegen. Zudem können die als Ausgangsstoffe eingesetzten Olefine entweder eine oder auch mehrere Kohlenstoff-Doppelbindungen enthalten.

Mit den erfindungsgemäßen Substanzen bzw. Katalysatoren können insbesondere die in diesem Text als bevorzugt benannten Olefine effektiv zu Aldehyden umgesetzt werden.

Die vorliegende Erfindung betrifft zudem ein Verfahren zur Herstellung von Aldehyden umfassend die Verfahrensschritte
a) Herstellen oder Bereitstellen eines Reaktionssystems umfassend
   a-1) eine erste Komponente umfassend ein oder mehrere Olefine mit jeweils einer oder mehreren Kohlenstoff-Doppelbindungen,
   a-2) eine Katalysatorkomponente umfassend
      - eine erfindungsgemäße Verbindung (vorzugsweise wie vorstehend als bevorzugt bezeichnet) und eines oder mehrere Metallatome,
   a-3) eine zweite Komponente umfassend H₂ und CO
b) Erwärmen des Reaktionssystems, sodass Kohlenstoff-Doppelbindungen des einen oder mindestens eines der mehreren Olefine zu Aldehyden umgesetzt werden.

Aus Olefinen können über Hydroformylierungsreaktionen Aldehyde gewonnen werden. In einer Hydroformylierungsreaktion findet formal die Addition eines Wasserstoffs (-H) und einer Formylgruppe (-CHO) an eine Kohlenstoff-Doppelbindung eines Moleküls statt; somit entstehen Aldehyde. Hierzu muss in Schritt a) ein Reaktionssystem hergestellt oder bereitgestellt werden.

Eine erste Komponente des Reaktionssystems a-1) umfasst eines oder mehrere Olefine mit jeweils einer oder mehreren Kohlenstoff-Doppelbindungen. Diese Komponente umfasst somit die Ausgangsstoffe bzw. Edukte, welche zu Aldehyden umgesetzt werden. Die eingesetzten Olefine können sowohl interne Kohlenstoff-Dippelbindungen (zum Beispiel 2-Penten), als auch endständige Kohlenstoff-Doppelbindungen (zum Beispiel 1,3-Butadien) haben. Zudem können die Olefine eine oder mehrere Kohlenstoff-Doppelbindungen enthalten.

Eine weitere Komponente des Reaktionssystems a-2), die sogenannte Katalysatorkomponente, umfasst eine erfindungsgemäße Verbindung (vorzugsweise wie vorstehend als bevorzugt bezeichnet) und eines oder mehrere Metallatome bzw. umfasst eine erfindungsgemäße Substanz. Die erfindungsgemäße Verbindung und das eine oder die mehreren Metallatome bilden den Katalysator der Hydroformylierungsreaktion. Werden die erfindungsgemäße Verbindung und das eine oder die mehreren Metallatome in Form eines Metallprecursors separat in das Reaktionssystem gegeben, so bildet sich der Katalysator *in situ.* Es können jedoch auch bereits isolierte Metallkomplexe direkt eingesetzt werden.

Eine zweite Komponente des Reaktionssystems a-3) umfasst H₂ und CO, auch Reaktivkomponente oder Synthesegas genannt. Die Herstellung von Aldehyden aus Olefinen wird formal durch Addition eines Wasserstoffs (-H) und einer Formylgruppe (-CHO) an eine Kohlenstoff-Doppelbindung gewährleistet. In der Praxis erfolgt die Umsetzung durch Zugabe von Wasserstoff (H₂) und Kohlenstoffmonoxid (CO) als Reaktivkomponente bzw. Synthesegas.

Durch das Erwärmen in Schritt b) findet die Reaktion statt und die Olefine werden zu den entsprechenden Aldehyden umgesetzt, wobei die Katalysatorkomponente die Reaktion beschleunigt.

Bevorzugt ist ein erfindungsgemäßes Verfahren, wobei das eine oder die mehreren Olefine ausgewählt ist/sind aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten.

Das erfindungsgemäße Verfahren eignet sich also zur Umsetzung einer Vielzahl an olefinischen Verbindungen.

Das erfindungsgemäße Verfahren und/oder der Einsatz erfindungsgemäßer Verbindungen ist zudem geeignet zur Herstellung von linearen unverzweigten n-Aldehyden aus Olefinen mit endständigen und innenliegenden Doppelbindungen, sogenannte n-Selektivität.

Für die Hydroformylierung gemäß dem erfindungsgemäßen Verfahren können verschiedene Übergangsmetalle, wie Rhodium, Cobalt, Eisen, Ruthenium, Osmium, Iridium und Platin als Metallatom verwendet werden. Auch Zinn ist als Metallatom geeignet.

Bevorzugt ist das eine oder eines der mehreren Metallatome im Rahmen des Verfahrens der vorliegenden Erfindung Cobalt oder Rhodium. Cobalt und Rhodium haben sich als Metallatom in Katalysatoren für die Hydroformylierung als besonders geeignet erwiesen.

Rhodiumkatalysatoren sind besonders effektiv in Bezug auf die Reaktionsgeschwindigkeit und den Umsatz und können bei niedrigeren Temperaturen und Drücken verwendet werden. Zudem ist Rhodium besonders kompatibel mit den hier definierten erfindungsgemäßen Verbindungen. Deshalb ist das eine oder eines der mehreren Metallatome deshalb besonders bevorzugt Rhodium.

Das Rhodium wird innerhalb des erfindungsgemäßen Verfahrens bevorzugt aus den kommerziell erhältlichen und gut handhabbaren Verbindungen Rh(acac)(CO)₂, [(acac)Rh(COD)] und/oder Rh₄CO₁₂ bereitgestellt. Diese Verbindungen werden auch Metallprecusoren bezeichnet.

In einer ersten besonders bevorzugten Ausgestaltung der vorliegenden Erfindung wird das Rhodium bereitgestellt aus der Verbindung [(acac)Rh(COD)]; diese Verbindung entspricht (Acetylacetonat)(1,5-cyclooctadien)rhodium(I) mit der CAS-Nr. 12245-39-5.

In einer zweiten besonders bevorzugten Ausgestaltung der vorliegenden Erfindung wird das Rhodium bereitgestellt aus der Verbindung Rh(acac)(CO)₂; diese Verbindung entspricht (Acetylacetonat)dicar-bonylrhodium(I) mit der CAS-Nr. 14874-82-9.

Bevorzugt ist ein erfindungsgemäßes Verfahren (vorzugsweise wie vorstehend als bevorzugt bezeichnet), wobei das molare Verhältnis von Metallatomen zu Verbindungen gemäß Formel (I) in der Katalysatorkomponente im Bereich von 1:0,8 bis 1:4 liegt, bevorzugt im Bereich von 1:0,9 bis 1:2,1 liegt, besonders bevorzugt 1:1 oder 1:2 ist.

Bevorzugt ist ein erfindungsgemäßes Verfahren (vorzugsweise wie vorstehend als bevorzugt bezeichnet), wobei die Katalysatorkomponente ein Metallkomplex ist oder einen Metallkomplex enthält, der Metallkomplex umfassend eine oder mehrere der erfindungsgemäßen Verbindungen.

Bevorzugt ist ein erfindungsgemäßes Verfahren (vorzugsweise wie vorstehend als bevorzugt bezeichnet), wobei die zweite Komponente umfassend H₂ und CO ein Gemisch aus H₂ und CO mit einem Stoffmengenverhältnis von H₂ zu CO im Bereich von 2:1 bis 1:2 ist, bevorzugt im Bereich von 1,5:1 bis 1:1,5, besonders bevorzugt im Bereich von 1,1:1 bis 1:1,1.

Formal entspricht diese Hydroformylierungsreaktion der Addition eines Wasserstoffs (-H) und einer Formylgruppe (-CHO) an eine Kohlenstoff-Doppelbindung. Hierfür werden in der Praxis in der Regel nahezu äquimolare Gemische von Wasserstoff (H₂) und Kohlenstoffmonoxid (CO) eingesetzt. Das besonders bevorzugte Stoffmengenverhältnis von H₂ zu CO liegt daher im Bereich von 1,1:1 bis 1:1,1, entspricht also einem ausgewogenen Verhältnis der Reaktivkomponenten.

Bevorzugt ist ein erfindungsgemäßes Verfahren (vorzugsweise wie vorstehend als bevorzugt bezeichnet), wobei das Reaktionssystem in Verfahrensschritt b) unter einem Druck im Bereich von 1 bis 30 bar steht, bevorzugt unter einem Druck im Bereich von 1,5 bar bis 25 bar steht.

Bevorzugt ist ein erfindungsgemäßes Verfahren (vorzugsweise wie vorstehend als bevorzugt bezeichnet), wobei das Reaktionssystem in Verfahrensschritt b) auf eine Temperatur im Bereich von 80 °C bis 160 °C erwärmt wird, bevorzugt auf eine Temperatur im Bereich von 100 °C bis 140 °C erwärmt wird.

Die vorgenannten bevorzugten Temperaturbereiche des Reaktionssystems in Verfahrensschritt b) erlauben eine effiziente und zugleich vergleichsweise schonende Reaktionsführung bei hohen Umsätzen.

Bevorzugt ist ein erfindungsgemäßes Verfahren (vorzugsweise wie vorstehend als bevorzugt bezeichnet), wobei das Reaktionssystem zusätzlich ein Lösungsmittel umfasst, wobei das Lösungsmittel vorzugsweise Toluol umfasst oder Toluol ist.

Bevorzugt ist ein erfindungsgemäßes Verfahren (vorzugsweise wie vorstehend als bevorzugt bezeichnet), wobei der Stoffmengenanteil von Rhodium im Reaktionssystem im Bereich von 10 µmol/mol bis 1000 µmol/mol liegt, bevorzugt im Bereich von 20 µmol/mol bis 500 µmol/mol liegt, besonders bevorzugt im Bereich von 50 µmol/mol bis 200 µmol/mol liegt, jeweils bezogen auf die Stoffmenge an Olefinen im Reaktionssystem.

Bevorzugt ist ein erfindungsgemäßes Verfahren (vorzugsweise wie vorstehend als bevorzugt bezeichnet), wobei ein Anteil von mindestens 50%, bevorzugt von mindestens 60%, besonders bevorzugt von mindestens 80%, der Kohlenstoff-Doppelbindungen des einen oder mindestens eines der mehreren Olefine umgesetzt wird.

Besonders bevorzugt ist ein erfindungsgemäßes Verfahren (vorzugsweise wie vorstehend als bevorzugt bezeichnet), wobei mindestens 85%, besonders bevorzugt mindestens 90%, ganz besonders bevorzugt mindestens 94% der Kohlenstoff-Doppelbindungen des einen oder mindestens eines der mehreren Olefine umgesetzt. Die eben genannten Vorteile sind bei diesen gesteigerten Umsätzen noch stärker ausgeprägt.

Bevorzugt ist ein erfindungsgemäßes Verfahren (vorzugsweise wie vorstehend als bevorzugt bezeichnet), umfassend die Verfahrensschritte
V1) Vorlegen eines Olefins;
V2) Zugabe einer erfindungsgemäßen Verbindung und einer Substanz welche Rhodium umfasst;
V3) Zuführen von H₂ und CO;
V4) Erwärmen des Reaktionssystems aus V1) bis V3), wobei das Olefin zu einem Aldehyd umgesetzt wird.

Die bevorzugte Abfolge des erfindungsgemäßen Verfahrens gemäß Verfahrensschritten V1) bis V4) hat sich als besonders effektiv und effizient erwiesen. Die Erfindung ist jedoch nicht auf die zuvor genannte Reihenfolge der Verfahrensschritte beschränkt.

Die vorliegende Erfindung betrifft zudem die Verwendung einer erfindungsgemäßen Verbindung (vorzugsweise wie vorstehend als bevorzugt bezeichnet) als Ligand eines Katalysators oder einer erfindungsgemäßen Substanz (vorzugsweise wie vorstehend als bevorzugt bezeichnet) als Katalysator in einer Hydroformylierungsreaktion.

Durch Verwendung der erfindungsgemäßen Verbindungen als Ligand eines Katalysators konnte in Hydroformylierungsreaktionen ein Umsatz von über 80 %, in einigen bevorzugten Ausgestaltungen von über 90%, in besonders bevorzugten Ausgestaltungen von über 94% erreicht werden. Des Weiteren zeigen die mit diesen Liganden hergestellten Katalysatoren die Eigenschaft, interne Olefine (zum Beispiel 2-Penten) in einem nicht unerheblichen Teil zu terminalen Aldehyden zu hydroformylieren, sogenannte n-Selektivität.

### BEISPIELE

Die folgenden Beispiele dienen der weiteren Erläuterung und Veranschaulichung der vorliegenden Erfindung, ohne deren Anwendungsbereich einzuschränken.

### 1) Synthese von erfindungsgemäßen Verbindungen:

### Verbindung I-i: 2,2'-((2,2-Dimethylpropan-1,3-diyl)bis(oxy))dinaphtho[1,8-de][1,3,2]dioxaphosphinin

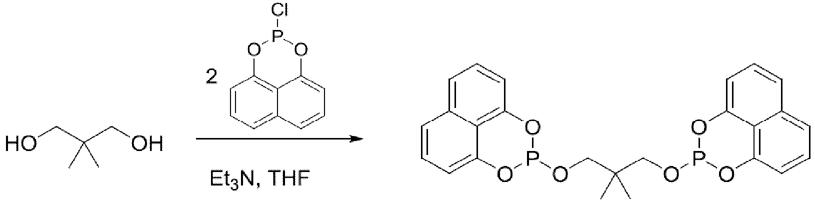

Eine Lösung mit 2,2-Dimethylpropan-1,3-diol (103 mg, 0,99 mmol) und Triethylamin (300 mg, 2,96 mmol) in THF (2 ml) wurde bei 0°C (Eisbad) vorgelegt und langsam mit 2-Chlornaphtho[1,8-*de*][1,3,2]dioxaphos-phinin (465 mg, 2,07 mmol) in THF (6 ml) versetzt. Die Mischung wurde anschließend über Nacht bei Raumtemperatur gerührt. Anschließend wurde das gebildete Hydrochlorid abfiltriert und das Lösungsmittel unter Vakuum entfernt. Der ölige Rückstand wurde bei 60°C getrocknet und nicht weiter aufgereinigt.

Ausbeute an Verbindung I-i: 380 mg farbloses Öl (80%).

³¹P-NMR (CD₂Cl₂): δ +112,2 ppm.

¹H-NMR (CD₂Cl₂): δ 7,51 (4H, dd, *J* 8,4, 0,9 Hz, 4xHₐᵣ), 7,39 (4H, dd, *J* 8,4, 7,4 Hz, 4xHₐᵣ), 6,89 (4H, dt, *J* 7,6, 0,9 Hz, 4xHₐᵣ), 3,45 (4H, d, *J* 7,5 Hz, 2xCH₂), 0,52 (6H, s, 2xCH₃).

¹³C-NMR (CDCl₃): δ 144,7 (d, 4xCₐᵣ-O), 135,4 (2xCₐᵣ), 127,6 (4xCₐᵣH), 122,3 (4xCₐᵣH), 117,6 (d, *J* 14,1 Hz, 2xCₐᵣ), 112,3 (4xCₐᵣH), 69,1 (d, *J* 13,0 Hz, 2xCH₂O), 36,7 (t, *J* 4,7 Hz, ^{t}C), 20,9 (2xCH₃).

HRMS (ESI-TOF) [M+H]⁺: *m*/*z* berechnet: für C₂₅H₂₂O₆P₂ 481,0970, gefunden: 481,0964.

| | | | | |
|---|---|---|---|---|
| Elementaranalyse: | berechnet: | C 62,51% | H 4,62% | P 12,89% |
| | gefunden: | C 62,35% | H 4,92% | P 12,79%. |

### Verbindung I-ii: 4,4'-((2,2-Dimethylpropan-1,3-diyl)bis(oxy))didinaphtho[2,1 d:1',2'-f][1,3,2]dioxaphosphe-pin

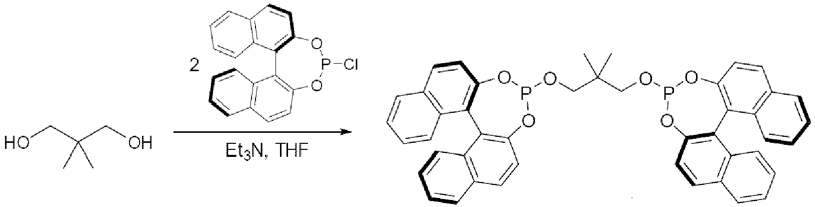

Eine Lösung mit 2,2-Dimethylpropan-1,3-diol (78 mg, 0,752 mmol) und Triethylamin (228 mg, 2,26 mmol) in THF (2 ml) wurde bei 0°C (Eisbad) vorgelegt und anschließend langsam mit dem chiralen (R)-4-chlor-dinaphtho[2,1-*d*:1',2'-*f*][1,3,2]dioxaphosphepin (554 mg, 1,58 mmol) in THF (6 ml) versetzt. Die Mischung wurde anschließend über Nacht bei Raumtemperatur gerührt. Daraufhin wurde das gebildete Hydrochlorid abfiltriert und anschließend das Lösungsmittel unter Vakuum entfernt. Der resultierende weiße Feststoff wurde getrocknet, mittels Säulenchromatographie (Eluent: Dichlormethan) vorgereinigt und abschließend aus Acetonitril umkristallisiert.

Ausbeute an Verbindung **I-ii:** 310 mg weißer Feststoff (56%).

³¹P-NMR (CD₂Cl₂): δ +141,9 ppm.

¹H-NMR (CD₂Cl₂): δ 7,99 (4H, m, 4xHₐᵣ), 7,90 (4H, m, 4xHₐᵣ), 7,49 (4H, dd, *J* 14,4, 8,8 Hz, 4xHₐᵣ), 7,46 (4H, m, 4xHₐᵣ), 7,37 (4H, m, 4xHₐᵣ), 7,29 (4H, ddd, *J* 8,6, 6,6, 1,3 Hz, 4xHₐᵣ), 3,80 (2H, dd, *J* 9,9, 6,7 Hz, 2xHₐ-CH₂), 3,62 (2H, dd, *J* 9,9, 6,1 Hz, 2xH_{b}-CH₂), 0,87 (6H, s, 2xCH₃).

¹³C-NMR (CD₂Cl₂): δ 149,3 (d, *J* 5,7 Hz, 2xCₐᵣ), 147,9 (2xCₐᵣ), 133,2 (2xCₐᵣ), 132,9 (2xCₐᵣ), 132,0 (2xCₐᵣ), 131,5 (2xCₐᵣ), 130,8 (2xCₐᵣH), 130,4 (2xCₐᵣH), 130,8 (2xCₐᵣH), 128,8 (4xCₐᵣH), 127,2 (4xCₐᵣH), 126,7 (2xCₐᵣH), 126,6 (2xCₐᵣH), 125,5 (2xCₐᵣH), 125,3 (2xCₐᵣH), 124,5 (d, *J* 5,4 Hz, 2xCₐᵣ), 122,9 (d, *J* 1,8 Hz, 2xCₐᵣ), 122,2 (2xCₐᵣH), 122,2 (2xCₐᵣH), 69,9 (d, *J* 6,3 Hz, 2xCH₂), 36,8 (t, *J* 4,5 Hz, ^{t}C), 21,3 (2xCH₃).

HRMS (ESI-TOF) [M+H]⁺: *m*/*z* berechnet: für C₄₅H₃₄O₆P₂ 733,1909, gefunden: 733,1915.

HRMS (ESI-TOF) [M+Na]⁺: *m*/*z* berechnet: für C₄₅H₃₄O₆P₂ 755,1722, gefunden: 733,1736.

| | | | | |
|---|---|---|---|---|
| Elementaranalyse: | berechnet: | C 73,77% | H 4,68% | P 8,45% |
| | gefunden: | C 73,92% | H 4,79% | P 8,20%. |

### Verbindung I-iii: 6,6'-((2,2-Dimethylpropan-1,3-diyl)bis(oxy))bis(4,8-di-tert-butyl-2,10-dimethoxydi-benzo[d,f][1,3,2]di-oxaphosphepin)

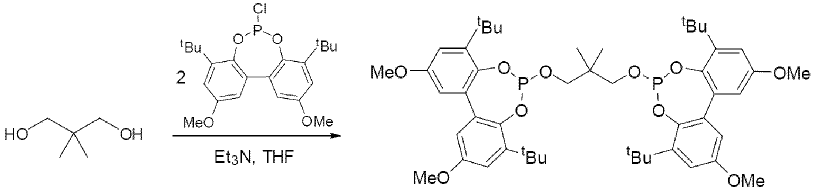

Eine Lösung mit 2,2-Dimethylpropan-1,3-diol (66 mg, 0,64 mmol) und Triethylamin (194 mg, 1,92 mmol) in THF (2 ml) wurde bei 0°C (Eisbad) vorgelegt und anschließend langsam mit dem 4,8-Di-*tert*-butyl-6-chloro-2,10-dimethoxydibenzo[*d*,*f*][1,3,2]dioxaphosphepin (554 mg, 1,58 mmol) in THF (6 ml) versetzt. Die Mischung wurde anschließend über Nacht bei Raumtemperatur gerührt. Das gebildete Hydrochlorid wurde abfiltriert und anschließend das Lösungsmittel unter Vakuum entfernt. Der so erhaltene weiße Feststoff wurde getrocknet und anschließend mittels Säulenchromatographie (Eluent: Dichlormethan) gereinigt.

Ausbeute an Verbindung **I-iii:** 388 mg weißer Feststoff (69%).

³¹P-NMR (CD₂Cl₂): δ +137,5 ppm.

¹H-NMR (CD₂Cl₂): δ 6,98 (4H, d, *J* 3,1 Hz, 4xHₐᵣ), 6,72 (4H, d, *J* 3,1 Hz, 4xHₐᵣ), 3,82 (12H, s, 4xCH₃O), 3,63 (4H, d, *J* 6,4 Hz, 2xCH₂O), 1,44 (36H, s, 4xC(CH₃)₃), 0,85 (6H, s, 2xCH₃).

¹³C-NMR (CD₂Cl₂): δ 156,1 (4xCₐᵣ-OMe), 142,7 (4xCₐᵣ), 142,5 (d, *J* 5,8 Hz, 4xCₐᵣ), 134,0 (d, *J* 3,6 Hz, 4xCₐᵣ), 114,7 (4xCₐᵣH), 113,2 (4xCₐᵣH), 69,7 (d, *J* 3,8 Hz, 2xCH₂), 56,0 (4xCH₃O), 37,1 (t, *J* 3,8 Hz, ^{t}C), 35,7 (4x^{t}c), 31,0 (d, *J* 2,5 Hz, 12xCH₃), 21,6 (2xCH₃).

HRMS (ESI-TOF) [M+H]⁺: *m*/*z* berechnet: für C₄₉H₆₆O₁₀P₂ 877,4210, gefunden: 877,4229.

HRMS (ESI-TOF) [M+Na]⁺: *m*/*z* berechnet: für C₄₉H₆₆O₁₀P₂ 899,4023, gefunden: 899,4041.

| | | | | |
|---|---|---|---|---|
| Elementaranalyse: | berechnet: | C67,11% | H 7,59% | P 7,06% |
| | gefunden: | C 67,19% | H 7,70% | P 7,14%. |

### Verbindung I-iv: 6,6'-((2,2-Dimethylpropane-1,3-diyl)bis(oxy))bis(2,4,8,10-tetra-tert.-butyldi-benzo[d,f][1,3,2]dioxa-phosphepin)

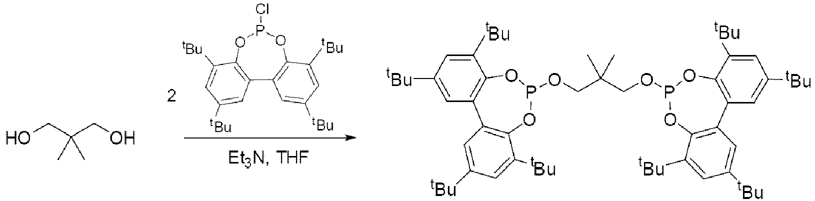

Eine Lösung mit 2,2-Dimethylpropan-1,3-diol (62 mg, 0,60 mmol) und Triethylamin (181 mg, 1,79 mmol) in THF (2 ml) wurde bei 0°C (Eisbad) vorgelegt und anschließend langsam mit dem 2,4,8,10-Tetra-tert-butyl-6-chlorodibenzo[*d*,*f*][1,3,2]dioxaphosphepin (594 mg, 1,25 mmol) in THF (6 ml) versetzt. Die Mischung wurde anschließend über Nacht bei Raumtemperatur gerührt. Daraufhin wurde das gebildete Hydrochlorid abfiltriert und anschließend das Lösungsmittel unter Vakuum entfernt. Der so erhaltene weiße Feststoff wurde getrocknet und anschließend mittels Säulenchromatographie (Eluent: Dichlormethan) gereinigt.

Ausbeute an Verbindung I-iv: 380 mg weißer Feststoff (65%).

³¹P-NMR (CD₂Cl₂): δ +139,1 ppm.

¹H-NMR (CD₂Cl₂): δ 7,46 (4H, d, *J* 2,5 Hz, 4xHₐᵣ), 7,19 (4H, d, *J* 2,5 Hz, 4xHₐᵣ), 3,59 (4H, d, *J* 6,4 Hz, 2xCH₂O), 1,47 (36H, s, 4xC(CH₃)₃), 1,38 (36H, s, 4xC(CH₃)₃), 0,82 (6H, s, 2xCH₃).

¹³C-NMR (CD₂Cl₂): δ 147,1 (4xCₐᵣ), 146,5 (d, *J* 6,1 Hz, 4xCₐᵣ), 140,5 (d, *J* 1,3 Hz, 4xCₐᵣ), 133,1 (d, *J* 3,7 Hz, 4xCₐᵣ), 126,8 (4xCₐᵣH), 124,8 (4xCₐᵣH), 69,7 (d, J 4,9 Hz, 2xCH₂), 37,0 (t, *J* 4,1 Hz, ^{t}C), 35,7 (4x^{t}C), 35,0 (4x^{t}C), 31,7 (12xCH₃), 31,3 (d, *J* 2,6 Hz, 12xCH₃), 21,6 (2xCH₃).

HRMS (ESI-TOF) [M+H]⁺: *m*/*z* berechnet: für C₆₁H₉₀O₆P₂ 981,6291, gefunden: 981,6317.

HRMS (ESI-TOF) [M+Na]⁺: *m*/*z* berechnet: für C₆₁H₉₀O₆P₂ 1003,6106, gefunden: 1003,6127.

| | | | | |
|---|---|---|---|---|
| Elementaranalyse: | berechnet: | C 74,66% | H 9,24% | P 6,31% |
| | gefunden: | C 74,61% | H 9,21% | P 6,50%. |

### Verbindung I-v-Di-Me: (4S,4'S,5R,5'R)-2,2'-((2,2-Dimethylpropan-1,3-diyl)bis(oxy))bis(4,5-diphenyl-1,3,2-dioxaphospholan)

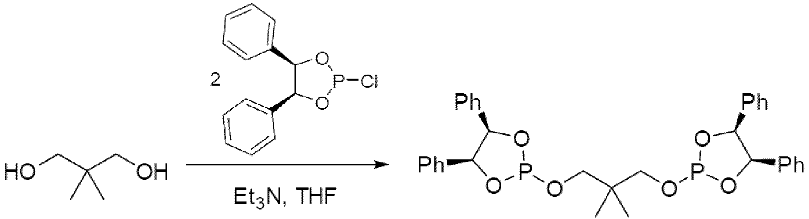

Das eingesetzte (4R,5S)-2-Chlor-4,5-diphenyl-1,3,2-dioxaphospholan wurde entsprechend der Literatur hergestellt (R. Kadyrov, D. Heller, R. Selke *Tetrahedron: Asymmetry* 1998, *9,* 329-340).

Eine Lösung mit 2,2-Dimethylpropan-1,3-diol (107 mg, 1,03 mmol) und Triethylamin (312 mg, 3,08 mmol) in THF (2 ml) wurde bei 0°C (Eisbad) vorgelegt und anschließend langsam mit dem (4*R*,5*S*)-2-Chlor-4,5-diphenyl-1,3,2-dioxaphospholan (602 mg, 2,16 mmol) in THF (4 ml) versetzt. Die Mischung wurde anschließend über Nacht bei Raumtemperatur gerührt. Daraufhin wurde das gebildete Hydrochlorid abfiltriert und anschließend das Lösungsmittel unter Vakuum entfernt. Der verbleibende weiße Feststoff wurde getrocknet, anschließend mit Acetonitril suspendiert und filtriert. Nach der Filtration wurde der Filterkuchen mit Acetonitril (5 ml) gewaschen und anschließend erneut getrocknet. Das Produkt wurde in einer Reinheit von ca. 95% (³¹P-NMR) erhalten und ohne weitere Reinigung weiterverwendet.

Ausbeute an Verbindung **I-v-Di-Me:** 245 mg weißer Feststoff (40%).

³¹P-NMR (CD₂Cl₂): δ +137,5 ppm.

¹H-NMR (CD₂Cl₂): δ 7,11-7,04 (12H, m, 12xHₐᵣ), 6,94-6,88 (8H, m, 8xHₐᵣ), 5,77 (4H, d, *J* 1,8 Hz, 4xCH-O), 3,82 (4H, d, *J* 7,8 Hz, 2xCH₂O), 1,05 (6H, s, 2xCH₃).

¹³C-NMR (CD₂Cl₂): δ 136,8 (d, *J* 4,0 Hz, 4xCₐᵣ), 128,0 (8xCₐᵣH), 127,9 (4xCₐᵣH), 127,2 (8xCₐᵣH), 82,1 (d, *J* 7,3 Hz, 4xCH-O), 68,9 (d, *J* 12,6 Hz, 2xCH₂O), 37,0 (d, *J* 4,3 Hz, ^{t}C), 21,7 (2xCH₃).

HRMS (ESI-TOF) [M+H]⁺: *m*/*z* berechnet: für C₃₃H₃₄O₆P₂ 589,1909, gefunden: 589,1918.

HRMS (ESI-TOF) [M+Na]⁺: *m*/*z* berechnet: für C₃₃H₃₄O₆P₂ 611,1722, gefunden:611,1738.

| | | | | |
|---|---|---|---|---|
| Elementaranalyse: | berechnet: | C 67,34% | H 5,82% | P 10,52% |
| | gefunden: | C 67,42% | H 5,68% | P 10,39%. |

### Verbindung I-v-Di-H: 1,3-Bis(((4S,5R)-4,5-diphenyl-1,3,2-dioxaphospholan-2-yl)oxy)propan

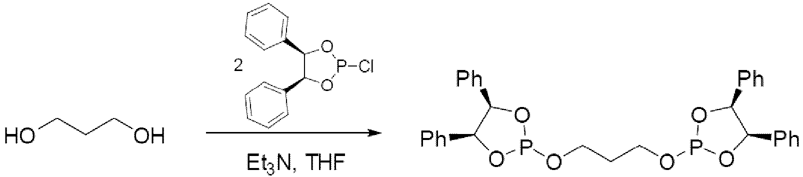

Eine Lösung mit Propan-1,3-diol (128 mg, 1,68 mmol) und Triethylamin (511 mg, 5,05 mmol) in THF (4 ml) wurde bei 0°C vorgelegt und anschließend langsam mit dem (4*R*,5S)-2-Chlor-4,5-diphenyl-1,3,2-dioxaphospholan (562 mg, 1,06 mmol) in THF (6 ml) versetzt. Die Mischung wurde anschließend über Nacht bei Raumtemperatur gerührt. Daraufhin wurde das Hydrochlorid abfiltriert und anschließend das Lösungsmittel unter Vakuum entfernt. Der verbleibende zähe Rückstand wurde bei 60°C unter Vakuum getrocknet und anschließend mit Heptan bei Siedehitze gelöst und heiß filtriert. Mit dem Abkühlen des Filtrats setzte sich am Boden eine leicht gelbe, ölige Phase ab, die beim Stehen im Tiefkühlschrank durch kristallisierte. Nach der Filtration und Trocknen des Feststoffs wurde das Produkt mit einer Reinheit von ca. 94% erhalten.

Ausbeute an Verbindung **I-v-Di-H:** 632 mg weißer Feststoff (67%).

³¹P-NMR (CD₂Cl₂): δ +137,5 ppm, zudem kleinere Signale bei +150,4 ppm und +137,4 ppm, bei denen es sich vermutlich um isomere Verbindungen handelt, welche aus der cis- und trans-Anordnungen der P-O-Bindung im Dioxophospholan resultieren.

¹H-NMR (CD₂Cl₂): δ 7,13-7,04 (12H, m, 12xHₐᵣ), 6,95-6,88 (8H, m, 8xHₐᵣ), 5,77 (4H, d, *J* 1,8 Hz, 4xCH-O), 4,37-4,14 (4H, dd, *J* 8,5, 6,1 Hz, 2xCH₂-O), 2,07 (2H, pent, *J* 6,1 Hz, CH₂).

¹³C-NMR (CD₂Cl₂): δ 136,7 (d, *J* 4,1 Hz, 2xCₐᵣ), 128,0 (4xCₐᵣH), 127,9 (2xCₐᵣH), 127,2 (4xCₐᵣH), 82,1 (d, *J* 7,3 Hz, 2xCH-O), 60,3 (d, *J* 13,9 Hz, 2xCH₂O), 33,2 (t, *J* 4,6 Hz, CH₂).

HRMS (ESI-TOF) [M+H]⁺: *m*/*z* berechnet: für C₃₁H₃₀O₆P₂ 561,1595, gefunden: 561,1599.

HRMS (ESI-TOF) [M+Na]⁺: *m*/*z* berechnet: für C₃₁H₃₀O₆P₂ 583,1409, gefunden: 583,1416.

| | | | | |
|---|---|---|---|---|
| Elementaranalyse: | berechnet: | C 66,43% | H 5,39% | P 11,05% |
| | gefunden: | C 66,55% | H 5,44% | P 11,24%. |

### Verbindung I-vi: (3aR,3a'R,8aR,8a'R)-6,6'-((2,2-Dimethylpropan-1,3-diyl)bis(oxy))bis(2,2-dimethyl-4,4,8,8-tetraphenyltetrahydro-[1,3]dioxolo[4,5-e][1,3,2]dioxaphosphepin)

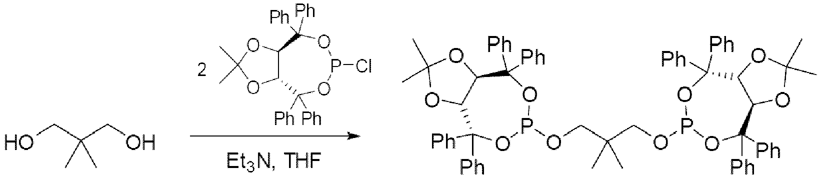

Das eingesetzte (3a*R*,8a*R*)-6-Chloro-2,2-dimethyl-4,4,8,8-tetraphenyltetrahydro-[1,3]dioxolo[4,5-e][1,3,2]dioxa-phosphepin wurde entsprechend der Literatur hergestellt (F. Blume, S. Zemolka, T. Fey, R. Kranich, H.-G. Schmalz Adv. Synth. Catal. 2002, 344, 868-883).

Eine Lösung mit 2,2-Dimethylpropan-1,3-diol (53 mg, 0,51 mmol) und Triethylamin (154 mg, 1,51 mmol) in THF (2 ml) wurde bei 0°C vorgelegt und anschließend langsam mit dem (3a*R*,8a*R*)-6-Chloro-2,2-dimethyl-4,4,8,8-tetraphenyltetrahydro-[1,3]dioxolo[4,5-e][1,3,2]dioxaphosphepin (562 mg, 1,06 mmol) in THF (4 ml) versetzt. Die Mischung wurde anschließend über Nacht bei Raumtemperatur gerührt. Daraufhin wurde das gebildete Hydrochlorid abfiltriert und anschließend das Lösemittel unter Vakuum entfernt. Der aus dem Filtrat verbleibende weiße Feststoff wurde getrocknet und anschließend mit Acetonitril in der Siedehitze gelöst. Das Produkt fiel daraufhin als weißer Feststoff aus. Nach dem Filtrieren wurde dieser unter Vakuum getrocknet.

Ausbeute an Verbindung **I-vi:** 375 mg weißer Feststoff (68%).

³¹P-NMR (CD₂Cl₂): δ +131,5 ppm.

¹H-NMR (CD₂Cl₂): δ 7,64-7,47 (16H, m, 16xHₐᵣ), 7,37-7,17 (24H, m, 24xHₐᵣ), 5,28 (2H, d, *J* 8,4 Hz, 2xCH-O), 5,03 (2H, dd, *J* 8,4, 1,3 Hz, 2xCH-O), 3,30 (4H, m, 2xCH₂O), 0,91 (6H, s, 2xCH₃), 0,69 (6H, s, 2xCH₃), 0,55 (6H, s, 2xCH₃).

¹³C-NMR (CD₂Cl₂): δ 147,0 (Cₐᵣ), 146,7(Cₐᵣ), 142,1 (Cₐᵣ), 142,1 (Cₐᵣ), 129,4 (4xCₐᵣH), 129,1 (4xCₐᵣH), 128,4 (4xCₐᵣH), 128,3 (4xCₐᵣH), 128,0 (2xCₐᵣH), 127,8 (6xCₐᵣH), 127,8 (6xCₐᵣH), 127,6 (2xCₐᵣH), 127,5 (8xCₐᵣH), 112,8 (^{t}CO₂), 84,6 (m, ^{t}C-O), 83,1 (m, ^{t}C-O), 83,0 (d, *J* 15,6 Hz, CH-O), 81,8 (m, CH-O), 68,4 (m, 2xCH₂O), 36,1 (t, *J* 5,1 Hz, ^{t}C), 26,9 (2xCH₃), 26,4 (2xCH₃), 21,2 (2xCH₃).

HRMS (ESI-TOF) [M+H]⁺: *m*/*z* berechnet: für C₆₇H₆₆O₁₀P₂ 1093,4209, gefunden: 1093,4222.

HRMS (ESI-TOF) [M+Na]⁺: *m*/*z* berechnet: für C₆₇H₆₆O₁₀P₂ 1115,4023, gefunden: 1115,4041.

| | | | | |
|---|---|---|---|---|
| Elementaranalyse: | berechnet: | C 73,61% | H 6,08% | P 5,67% |
| | gefunden: | C 73,80% | H 6,34% | P 5,45%. |

### 2) Synthese von nicht-erfindungsgemäßen Vergleichsverbindungen:

### Verbindung NE-1: ((2,2-Dimethylpropan-1,3-diyl)bis(oxy))bis(di-o-tolylphosphan)

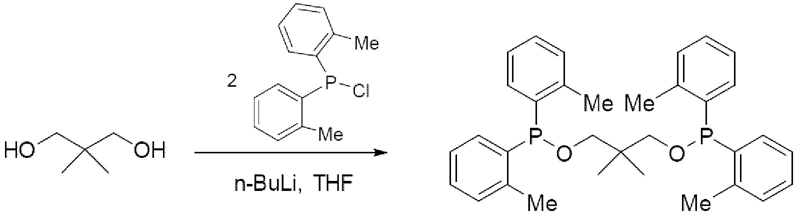

Eine Lösung des 2,2-Dimethylpropan-1,3-diols (87 mg, 0,84 mmol) in THF (2 ml) wurde auf -20°C abgekühlt und langsam mit n-Butyllithium in Hexan (1,6M, 1,1 ml, 1,76 mmol) versetzt. Nach etwa 20 Minuten wurde zu der trüben Lösung das Chlor(di-o-tolyl)phosphan (438 mg, 1,76 mmol) in THF (5 ml) bei Raumtemperatur zugegeben. Die Mischung wurde anschließend über Nacht bei Raumtemperatur gerührt. Daraufhin wurde das Lösungsmittel unter Vakuum entfernt und der Rückstand mit Toluol (5 ml) aufgenommen. Nach der Filtration wurde der viskose Rückstand unter Vakuum bei 60°C getrocknet. Die Reinigung des Bisphosphinits erfolgte mittels Säulenchromatographie (Eluent: Heptan/Dichlormethan 1/2).

Ausbeute an **NE-1:** 250 mg zäher Sirup (56%).

³¹P-NMR (CD₂Cl₂): δ +101,3 ppm (Produkt).

¹H-NMR (CD₂Cl₂): δ 7,45 (4H, m, 4xHₐᵣ), 7,27 (4H, m, 4xHₐᵣ), 7,22-7,13 (8H, m, 8xHₐᵣ), 3,65 (4H, d, *J* 6,9 Hz, 2xCH₂O), 2,41 (12H, s, 4xCH₃), 0,96 (6H, s, 2xCH₃).

¹³C-NMR (CD₂Cl₂): δ 141,3 (d, *J* 25,8 Hz, 4xCₐᵣ), 139,7 (d, *J* 20,5 Hz, 4xCₐᵣ), 130,7 (d, *J* 6,9 Hz, 4xCₐᵣH), 130,4 (d, *J* 4,2 Hz, 4xCₐᵣH), 129,6 (4xCₐᵣH), 126,2 (d, J 2,4 Hz, 4xCₐᵣH), 76,1 (d, *J* 18,9 Hz, 2xCH₂O), 38,5 (t, *J* 8,6 Hz, ^{t}C), 22,0 (2xCH₃), 20,8 (d, *J* 20,3 Hz, 4xCH₃).

HRMS (ESI-TOF) [M+H]⁺: *m*/*z* berechnet: für C₃₃H₃₈O₂P₂ 529,2425, gefunden: 529,2415.

HRMS (ESI-TOF) [M+O+H]⁺: *m*/*z* berechnet: für C₃₃H₃₈O₂P₂ 545,2374, gefunden: 545,2362.

| | | | | |
|---|---|---|---|---|
| Elementaranalyse: | berechnet: | C 74,66% | H 9,24% | P 6,31% |
| | gefunden: | C 74,61% | H 9,21% | P 6,50%. |

### Verbindung NE-2:

### ((2,2-Dimethylpropan-1,3-diyl)bis(oxy))bis(dicyclohexylphosphan)

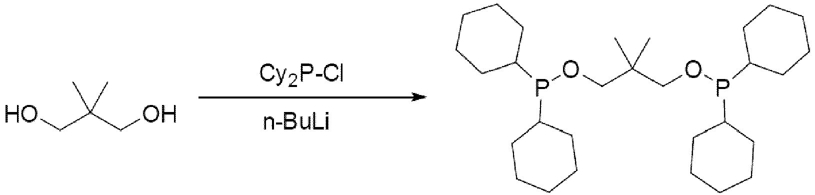

Eine Lösung des 2,2-Dimethylpropan-1,3-diol (146 mg, 1,40 mmol) in THF (3 ml) wurde bei -20°C langsam mit einer *n*-Butyllhitium-Lösung (1,6 M in Hexan, 1,84 ml, 2,94 mmol) versetzt. Nach ca. 20 Minuten wurde die Mischung man auf Raumtemperatur erwärmt und anschließend eine Chlordicyclohexylphosphan-Lösung (684 mg, 2,94 mmol) in THF (5 ml) hinzugegeben. Nach dem Rühren über Nacht wurde das Lösungsmittel entfernt und der Rückstand mit Toluol (6 ml) aufgenommen. Die unlöslichen Bestandteile wurden anschließend abfiltriert und das Toluol unter Vakuum aus dem Filtrat entfernt. Der viskose Rückstand wurde bei 60°C getrocknet und anschließend mit wenig THF gelöst und durch Zugabe von Acetonitril zur Kristallisation gebracht. Nach Filtration und Trocknen wurde das Bisphosphinit mit hoher Reinheit erhalten.

Ausbeute an **NE-2:** 400 mg weißer Feststoff (57%).

³¹P-NMR (CD₂Cl₂): δ +145,9 ppm.

¹H-NMR (CD₂Cl₂): δ 3,39 (4H, d, *J* 5,5 Hz, 2xCH₂O), 1,93-1,13 (44H, m, 4xCH + 20xCH₂), 0,87 (6H, s,2xCH₃).

¹³C-NMR (CD₂Cl₂): δ 78,4 (d, *J* 17,6 Hz, 2xCH₂O), 39,1 (t, *J* 8,3 Hz, ^{t}C), 38,6 (d, *J* 17,5 Hz, 4xCH-P), 28,9 (d, *J* 18,9 Hz, 4xCH₂), 27,6 (d, *J* 2,0 Hz, 4xCH₂), 27,4 (4xCH₂), 27,4 (d, *J* 11,4 Hz, 4xCH₂), 27,1 (4xCH₂), 22,2 (2xCH₃).

HRMS (ESI-TOF) [M+2O+H]⁺: *m*/*z* berechnet: für C₂₅H₅₄O₂P₂ 529,3575, gefunden: 529,3580.

HRMS (ESI-TOF) [M+2O+Na]⁺: *m*/*z* berechnet: für C₂₅H₅₄O₂P₂ 551,3389, gefunden: 551,3397.

| | | | | |
|---|---|---|---|---|
| Elementaranalyse: | berechnet: | C 70,13% | H 10,96% | P 12,47% |
| | gefunden: | C 70,14% | H 10,83% | P 12,29%. |

### 3) Einsatz erfindungsgemäßer Verbindungen und Substanzen in Hydroformylierungsreaktionen

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung des Einflusses von Feuchtigkeit und Sauerstoff wurde das als Solvens benutzte Toluol in einem Pure Solv. MD-7 System gereinigt und unter Argon aufbewahrt. Cis/trans-2-Penten (Aldrich) wurde über Natrium am Rückfluss erhitzt und unter Argon destilliert. Im Autoklav wurden unter Argonatmosphäre Lösungen der Katalysatorvorstufe (Metallprecursor) und der erfindungsgemäßen bzw. nicht-erfindungsgemäßen Verbindungen als Liganden jeweils in Toluol gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur wurde das in der Druckpipette befindliche Olefin mit einem Überdruck von ca. 3 bar in den Autoklaven gepresst. Die Reaktion wurde bei konstantem Druck (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischung wurden entnommen, mit 10 ml Pentan verdünnt und mittels Gaschromatographie analysiert: HP 5890 Series II plus, PONA, 50 m x 0,2 mm x 0,5 µm.

Die in Tabelle 1 dargestellten Ergebnisse der Hydroformylierung wurden unter folgenden Bedingungen / Parametern erhalten:
- eingesetztes Olefin: 2-Penten (2,41 M),
- Temperatur: 120°C,
- Reaktionszeit: 4 h,
- Druck: 20 bar,
- Zusammensetzung des Synthesegases: CO/H₂ (1:1),
- Lösungsmittel: Toluol,
- eingesetzte Menge an Rhodium in Form von [(acac)Rh(COD)] bezogen auf die eingesetzte Menge an 2-Penten: µmol/mol,
- molares Verhältnis von Rhodiumatomen zu eingesetzter Verbindung 1:2.

**Tabelle 1. Ergebnisse in der Hydroformylierung von 2-Penten**

| Verbindung | Umsatz (%) | n-Selektivität (%) |
|---|---|---|
| I-i | 82 | 33,7 |
| I-ii | 99 | 38,8 |
| I-iii | 90 | 40,1 |
| I-iv | 85 | 46,3 |
| I-v-Di-Me | >99 | 26,1 |
| I-v-Di-H | >99 | 27,5 |
| I-vi | 94 | 22,7 |
| NE-1 | 20 | 40,7 |
| NE-2 | 0 | - |

Anhand der in Tabelle 1 dargestellten Ergebnisse konnte gezeigt werden, dass die erfindungsgemäßen Verbindungen und entsprechende Substanzen sowie auch das erfindungsgemäße Verfahren in der Hydroformylierung von Olefinen, hier beispielhaft gezeigt an dem Substrat 2-Penten, hervorragende Resultate zeigen.

Sämtliche der eingesetzten erfindungsgemäßen Verbindungen I-i, I-ii, I-iii, I-iv, I-v, I-v-Di-Me, I-v-Di-H und I-vi zeigten exzellent hohe Umsätze von ≥82% bei der Hydroformylierung von 2-Penten in die entsprechenden Aldehyde.

Die Umsätze bei entsprechendem katalytischem Einsatz der erfindungsgemäßen Verbindungen zur Hydroformylierung von 2-Penten in die entsprechenden Aldehyde liegt in Teilen deutlich über 90%, vgl. insbesondere die Performance der Verbindungen I-ii, I-v-Di-Me, I-v-Di-H und I-vi.

Die nicht-erfindungsgemäßen Vergleichsverbindungen NE-1 und NE-2 hingegen zeigen entweder gar keine katalytische Aktivität bei der Hydroformylierung von 2-Penten, vgl. den Umsatz von 0% für NE-2, oder nur eine sehr geringe katalytische Aktivität, vgl. den Umsatz von 20% für NE-1.

Im Rahmen der vorliegenden Erfindung ist es somit gelungen durch gezielte Wahl der spezifischen Strukturelemente (i), (ii), (iii), (iv), (v) und (vi) in Verbindung mit der in den Ansprüchen und der Beschreibung definierten allgemeinen Verbindung gemäß Formel (I) bisher unbekannte Organphosphit-Liganden als Basis für Katalysatoren in der Hydroformylierung bereitzustellen, welche vorteilhaft hohe Umsätze erzielen.

Hierbei zeigte sich auch, dass die katalytische Aktivität der eingesetzten Verbindungen in der Hydroformylierung insbesondere in Kombination mit Rhodium-Metallzentren besonders effektiv ist und so exzellente Umsätze erzielt werden können.

Des Weiteren zeigen die Ergebnisse, dass die erfindungsgemäßen Verbindungen I-i, I-ii, I-iii, I-iv, I-v, I-v-Di-Me, I-v-Di-H und I-vi ebenfalls dazu in der Lage sind, gute Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier untersuchten 2-Penten der Fall, eine besonders schwer einzustellende Größe ist. Folglich sind die mit den erfindungsgemäßen Verbindungen I-i, I-ii, I-iii, I-iv, I-v, I-v-Di-Me, I-v-Di-H und I-vi erzielten n-Selektivitäten, d.h. die Selektivität für das Produkt Hexanal, im Bereich von 22 bis 47% bereits sehr gute Resultate. Insbesondere die Verbindungen (I-ii), (I-iii) und (I-iv) zeigten hierbei sehr gute n-Selektivitäten von mehr als 38% und bis zu 46,3%.

## Patentansprüche

1. Verbindung gemäß Formel (I), 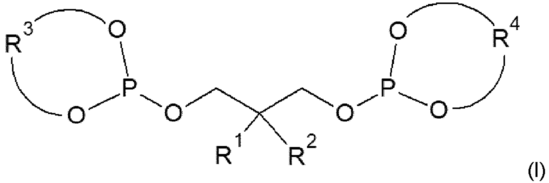 wobei
- R¹ und R² jeweils unabhängig voneinander ausgewählt sind aus: H und CH₃
und
- R³ und R⁴ jeweils unabhängig voneinander ausgewählt sind aus den Strukturelementen (i), (ii), (iii), (iv), (v) und (vi)

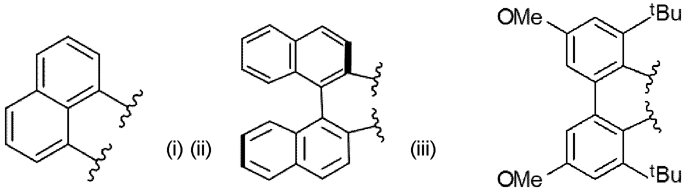

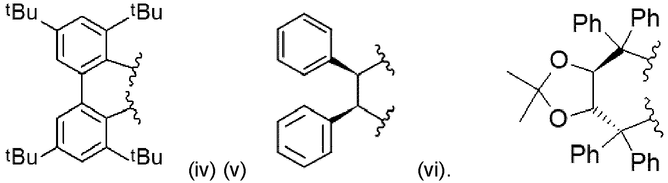

2. Verbindung nach Anspruch 1, wobei
- R¹ und R² jeweils H sind,
oder
- R¹ und R² jeweils CH₃ sind.

3. Verbindung nach einem der vorherigen Ansprüche, wobei R³ und R⁴ identisch sind und jeweils
- das Strukturelement (i) sind,
oder
- das Strukturelement (ii) sind,
oder
- das Strukturelement (iii) sind,
oder
- das Strukturelement (iv) sind,
oder
- das Strukturelement (v) sind,
oder
- das Strukturelement (vi) sind.

4. Verbindung nach einem der vorherigen Ansprüche, ausgewählt aus der Gruppe von Verbindungen mit den folgenden Formeln (I-i), (I-ii), (I-iii), (I-iv), (I-v), (I-v-Di-Me), (I-v-Di-H), oder (I-vi): 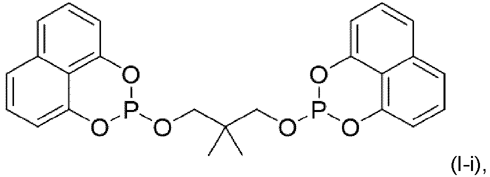 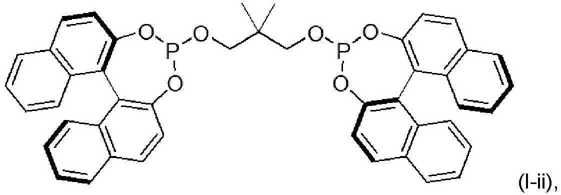 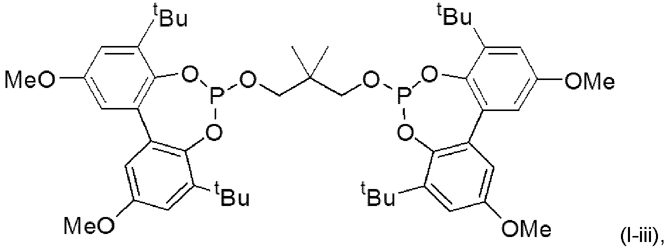 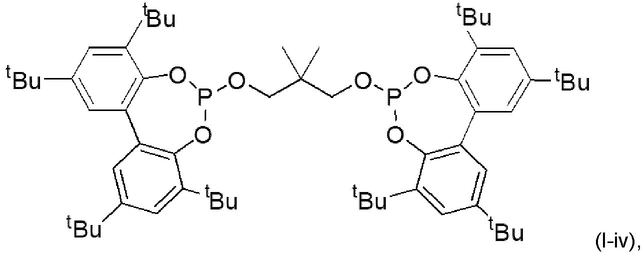 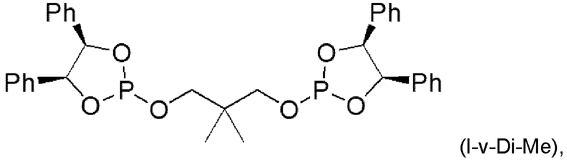 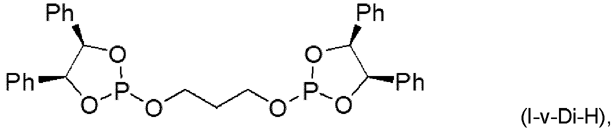 oder

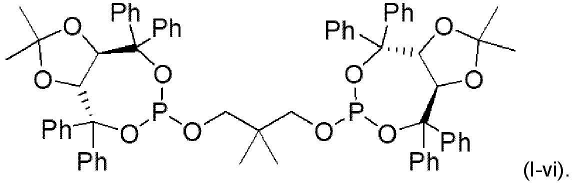

5. Substanz, wobei die Substanz
i) eine Mischung ist, die Mischung umfassend
- eine oder mehrere der Verbindungen gemäß Formel (I) nach einem der Ansprüche 1 bis 4,
und
- eines oder mehrere Metallatome,
und/oder
ii) ein Metallkomplex ist oder einen Metallkomplex enthält, der Metallkomplex umfassend
- eine oder mehrere der Verbindungen gemäß Formel (I) nach einem der Ansprüche 1 bis 4,
und
- eines oder mehrere Metallatome.

6. Substanz nach Anspruch 5, wobei
- das molare Verhältnis von Metallatomen zu Verbindungen gemäß Formel (I) in der Mischung und/oder im Metallkomplex im Bereich von 0,5:1 bis 1:4 liegt,
bevorzugt im Bereich von 0,9:1 bis 1:2,1 liegt, besonders bevorzugt 1:1 oder 1:2 ist,
und/oder
- das eine oder eines der mehreren Metallatome in der Mischung und/oder im Metallkomplex ausgewählt ist aus der Gruppe bestehend aus Rhodium, Cobalt, Eisen, Ruthenium, Osmium, Iridium, Platin und Zinn,
bevorzugt ist das eine oder eines der mehreren Metallatome Rhodium,
und/oder
- die Mischung als weiteren Bestandteil umfasst
- ein oder mehrere Olefine mit jeweils einer oder mehreren Kohlenstoff-Doppelbindungen,
bevorzugt ausgewählt aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
und/oder
- Lösungsmittel,
wobei das Lösungsmittel vorzugsweise Toluol umfasst oder Toluol ist.

7. Verfahren zur Herstellung von Aldehyden umfassend die Verfahrensschritte
a) Herstellen oder Bereitstellen eines Reaktionssystems umfassend
a-1) eine erste Komponente umfassend ein oder mehrere Olefine mit jeweils einer oder mehreren Kohlenstoff-Doppelbindungen,
a-2) eine Katalysatorkomponente umfassend
- eine Verbindung gemäß einem der Ansprüche 1 bis 4 und eines oder mehrere Metallatome,
a-3) eine zweite Komponente umfassend H₂ und CO
b) Erwärmen des Reaktionssystems, sodass Kohlenstoff-Doppelbindungen des einen oder mindestens eines der mehreren Olefine zu Aldehyden umgesetzt werden.

8. Verfahren nach Anspruch 7, wobei das eine oder die mehreren Olefine ausgewählt ist/sind aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten.

9. Verfahren nach einem der Ansprüche 7 oder 8, wobei
- das eine oder eines der mehreren Metallatome der Katalysatorkomponente ausgewählt ist aus der Gruppe bestehend aus Rhodium, Cobalt, Eisen, Ruthenium, Osmium, Iridium, Platin und Zinn,
bevorzugt ist das eine oder eines der mehreren Metallatome Rhodium,
besonders bevorzugt wird das Rhodium bereitgestellt aus einer der folgenden Verbindungen: Rh(acac)(CO)₂, [(acac)Rh(COD)] und Rh₄CO₁₂,
und/oder
- das molare Verhältnis von Metallatomen zu Verbindungen gemäß Formel (I) in der Katalysatorkomponente im Bereich von 1:0,8 bis 1:4 liegt,
bevorzugt im Bereich von 1:0,9 bis 1:2,1 liegt,
besonders bevorzugt 1:1 oder 1:2 ist,
und/oder
- die Katalysatorkomponente ein Metallkomplex ist oder einen Metallkomplex enthält, der Metallkomplex umfassend eine oder mehrere der Verbindungen nach einem der Ansprüche 1 bis 4 und Rhodium.

10. Verfahren nach einem der Ansprüche 7 bis 9, wobei die zweite Komponente umfassend H₂ und CO ein Gemisch aus H₂ und CO mit einem Stoffmengenverhältnis von H₂ zu CO im Bereich von 2:1 bis 1:2 ist, bevorzugt im Bereich von 1,5:1 bis 1:1,5, besonders bevorzugt im Bereich von 1,1:1 bis 1:1,1.

11. Verfahren nach einem der Ansprüche 7 bis 10, wobei das Reaktionssystem in Verfahrensschritt b)
- unter einem Druck im Bereich von 1 bis 30 bar steht, bevorzugt unter einem Druck im Bereich von 1,5 bar bis 25 bar steht,
und/oder
- auf eine Temperatur im Bereich von 80 °C bis 160 °C erwärmt wird, bevorzugt auf eine Temperatur im Bereich von 100 °C bis 140 °C erwärmt wird.

12. Verfahren nach einem der Ansprüche 7 bis 11, wobei
- das Reaktionssystem zusätzlich ein Lösungsmittel umfasst, wobei das Lösungsmittel vorzugsweise Toluol umfasst oder Toluol ist,
und/oder
- der Stoffmengenanteil von Rhodium im Reaktionssystem im Bereich von 10 µmol/mol bis 1000 µmol/mol liegt, bevorzugt im Bereich von 20 µmol/mol bis 500 µmol/mol liegt, besonders bevorzugt im Bereich von 50 µmol/mol bis 200 µmol/mol liegt, jeweils bezogen auf die Stoffmenge an Olefinen im Reaktionssystem,
und/oder
- wobei das Stoffmengenverhältnis von Rhodium zu den Verbindungen gemäß einem der Ansprüche 1 bis 4 in der Katalysatorkomponente im Bereich von 1:1 bis 1:3 liegt, bevorzugt im Bereich von 1:1,5 bis 1:2,5 liegt, besonders bevorzugt im Bereich von 1:1,9 bis 1:2,1 liegt.

13. Verfahren nach einem der Ansprüche 7 bis 12, wobei ein Anteil von mindestens 50%, bevorzugt von mindestens 60%, besonders bevorzugt von mindestens 80%, der Kohlenstoff-Doppelbindungen des einen oder mindestens eines der mehreren Olefine umgesetzt wird.

14. Verfahren nach einem der Ansprüche 7 bis 13, umfassend die Verfahrensschritte
V1) Vorlegen eines Olefins;
V2) Zugabe einer Verbindung gemäß einem der Ansprüche 1 bis 4 und einer Substanz welche Rhodium umfasst;
V3) Zuführen von H₂ und CO;
V4) Erwärmen des Reaktionssystems aus V1) bis V3), wobei das Olefin zu einem Aldehyd umgesetzt wird.

15. Verwendung einer Verbindung nach einem der Ansprüche 1 bis 4 als Ligand eines Katalysators oder einer Substanz nach einem der Ansprüche 5 oder 6 als Katalysator in einer Hydroformylierungsreaktion, bevorzugt in einer Hydroformylierungsreaktion gemäß einem Verfahren wie in den Ansprüchen 7 bis 14 definiert.
